# EUROPEAN PATENT SPECIFICATION

(11) **EP 0 782 459 B1**
(45) Date of publication and mention of the grant of the patent: **02.04.2003**
(21) Application number: 95937279.8
(22) Date of filing: 13.11.1995
(51) Int. Cl.: A61M 1/14, A61M 1/16, A61M 5/145

(54) **A MICRODIALYSIS DEVICE**
EINE MIKRODIALYSEVORRICHTUNG
DISPOSITIF DE MICRODIALYSE

(30) Priority: 14.11.1994 SE 9403909
(43) Date of publication of application: 09.07.1997
(73) Proprietor: CMA/MICRODIALYSIS AB, 104 05 Stockholm (SE)
(72) Inventor: UNGERSTEDT, Urban, S-181 46 Lidingö (SE); KARLSSON, Hans, S-191 44 Sollentuna (SE)
(74) Representative: Grennberg, Erik Bertil
(86) International application number: SE9501343
(87) International publication number: WO96014889

(56) References cited:
- WO-A-89/02720
- WO-A-90/14791
- WO-A-92/18191
- AU-B- 607 982

## Description

The present invention relates to a microdialysis device of the kind which includes an elongated microdialysis catheter having a semi-permeable membrane included in a container, which together form an outer tube surrounding an inner tube, which has an opening at the distal end of the catheter, and a first line is connected to the proximal end of the inner tube and a second line is connected to the proximal end of a space formed between the inner tube and the outer tube, a pump for delivering dialysis liquid connected to one of the lines, and means for collecting through-pumped dialysis liquid from the free end of the second line. The invention also relates to a pump suitable for use with the device, and to a method suited for its operation.

A microdialysis catheter of this kind can be used for the in vivo performance of analyses of the different molecules contained in intercellular body fluids and capable of diffusing through the semi-permeable membrane. Microdialysis catheters can also be used to deliver different molecules via the dialysis liquid supplied.

A microdialysis catheter of the aforedescribed kind is known from SE-C-434 214. Small pumps for driving dialysis liquid through a catheter of this kind are also known to the art. These pumps normally include a cylinder and plunger of the kind used with injection syringes, and a drive means and motor for moving the plunger forwards in the cylinder. Such pump arrangements normally work continuously, although insulin pumps in particular may work intermittently, wherein insulin is delivered periodically and the supply of insulin is controlled by varying the amount delivered each time.

A microdialysis device comprising the features defined in the preamble of claim 1 is known from document WO-A-90/14791.

A particular feature with regard to microdialysis resides in the extremely small quantities of liquid required to fill a microdialysis catheter, namely quantities in the order of less than 1 microliter, for instance 0.5 microliter, when compared with the fact that a droplet pouring freely from a narrow pipe is of the order of 15 microlitres.

In the case of stationary systems, it is normal and practical to work with continuous dialysis liquid throughflow. In the case of transportable systems, and particularly systems carried by a patient, which must be battery-operated, the requirement of low current consumption makes the use of intermittently operating pumps preferable. However, it has been found that commercially available apparatus are liable to provide excessively large quantities of sample fluid, in which the substances to be analyzed are lowly concentrated. Commercially available insulin pumps have also been found less suitable, because they are difficult and troublesome to handle. An object of the invention is therefore to provide an improved system, particularly with regard to the actual pump arrangement.

This and other objects are achieved in accordance with the invention by virtue of the pump of a system of the kind defined in the introduction is adapted to deliver intermittently on each intermittent pumping occasion a predetermined fluid quantity which is at most equal to roughly the volume that can be taken-up in the space between the inner tube and the outer tube over a length thereof that corresponds to the extension of the semi-permeable membrane in the longitudinal direction of the microdialysis catheter. This fluid volume will be in the order of 0.5 microliter in the case of a typical microdialysis catheter.

This results in improved efficiency, since only fluid that has been located close to the semi-permeable membrane will be transported and replaced on each intermittent occasion. If a large quantity of fluid is replaced on each occasion, part of the outflowing fluid will never have been located in the zone in which exchange through the semi-permeable membrane occurs, meaning that the fluid volume of interest from an analysis aspect will be unnecessarily diluted with fluid that lacks the factor to be analyzed. Using a signal analysis analogy, this means that the signal to noise ratio will be impaired.

The pump will also preferably have the possibility of executing an initial flushing process, in which all fluid in tube and catheter will be replaced, normally to an amount of roughly 100 µl. Optionally, this may be effected manually, prior to fitting a pump chamber and plunger constructed as an injection syringe into a motorized drive means, together forming a pump operative in pumping dialysis fluid through the microdialysis device. According to a particularly advantageous embodiment, the drive means has the form of a casing which accommodates the pump chamber and plunger and which is coupled to a battery-operated electric motor by means of a threaded screw, wherein the plunger has an extension externally of the pump chamber which has a perpendicularly disposed open bifurcate form which can be resiliently clamped about the screw, wherein at least one of the inwardly facing sides of the bifurcate form has a pattern of furrows or grooves whose periodicity coincides essentially with the pitch of the thread of the screw and a slope in relation to the direction of plunger movement which corresponds to the slopes of the threads on one side thereof against which the inwardly facing grooved side of the bifurcate form is resiliently disposed. In order to avoid the need to centre accurately, a particular advantage is afforded when only one side is provided with a screw-thread. The bifurcate form is preferably made of plastic, such as Delrin. The screw is preferably made of metal, conveniently of steel.

The invention will now be described with reference to a nonlimiting embodiment thereof and also with reference to the accompanying drawings.

Fig. 1 illustrates schematically a microdialysis device with catheter, pump and sample-collecting means.

Fig. 2 illustrates a microdialysis device fitted to a patient.

Fig. 3 is a block diagram illustrating a microdialysis pump.

Fig. 4 is a sectional view of a casing with drive means, for housing a pump chamber of the injection syringe kind and corresponding to a design illustrated in Fig. 3.

Fig. 5 illustrates a running nut which is intended to be fitted to the plunger rod of an injection syringe.

Fig. 6 is a cross-sectional view taken on the line V-V in Fig. 5.

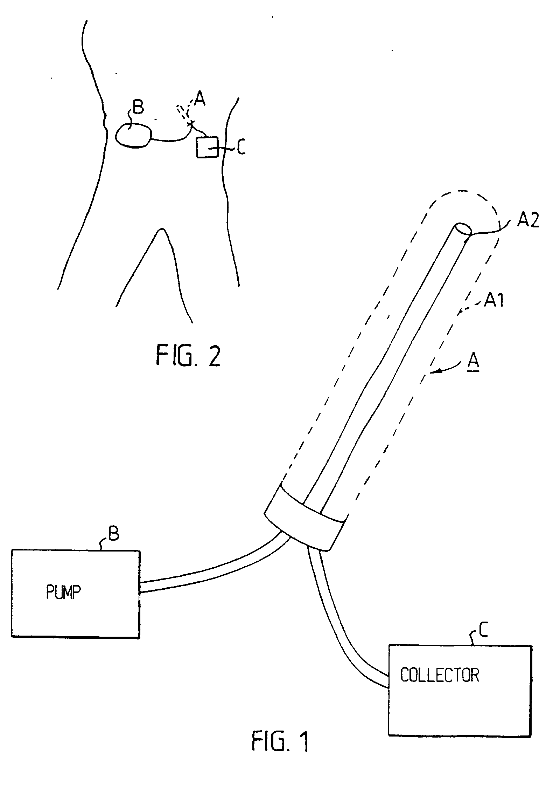

Fig. 1 is a highly schematic illustration of a microdialysis device having a catheter A which is supplied with fluid by a pump B and which delivers fluid to a collector C. A semi-permeable membrane A1 forms an outer casing which surrounds a tube A2, and the tube A2 and the space defined between the tube A2 and the membrane A1 are each connected to a respective supply and drain tube connected to pump B and collector C respectively. The catheter A is normally inserted into tissue, as shown in Fig. 2, and a pump B according to the invention is attached to the patient's body, as is also a schematically indicated collector C.

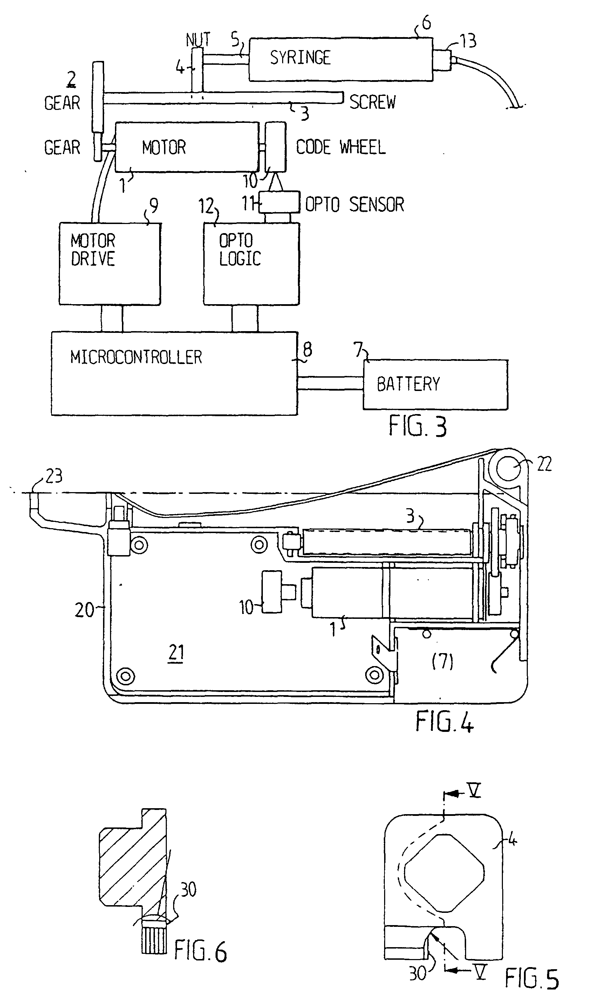

The principle illustration in Fig. 3 shows the active components of a microdialysis pump, and a drive motor 1 which drives a screw 3 through the medium of a transmission 2. The screw is straddled by an open nut 4, as described in more detail below, which is fastened to (or made integral with) the plunger rod of a pump corresponding to a syringe or the like 6. The power source has the form of a battery which is controlled by a microcontroller 8 to deliver electric current to the motor 1, via a motor drive unit 9. The motor has a through-extending shaft and carries on the end thereof distal from the transmission gearing a code wheel 10 provided with dark and light stripes (not shown) which are sensed by an opto-sensor 11 (light source and light sensor in combination), which signals rotation of the motor to the microcontroller 8 via an opto-logic means.

As will be seen from the cut-away view shown in Fig. 4, the motor 1, the code wheel 10, the gearing and the screw 3 and the battery (7) are all neatly mounted in a housing 20. Remaining components are mounted on the circuit card 21 indicated in Fig. 4. Although not shown, an upper part is pivotally mounted at 22, wherein an injection syringe of the type intended for one-time use only and provided with a "straddle nut" 4 can be fitted and clamped firmly beneath the lid with the outlet end 13 of the syringe lying in an opening 23 and the plunger rod connected for translatory movement upon rotation of the screw 3.

This simple arrangement is made possible by using a nut 4, shown in more detail in Figs. 5 and 6, which includes a U-shaped recess whose one U-leg has a screw-thread 30 cut thereon, wherein the distance between the legs is adapted to the screw-thread on the screw 3.

In the case of one example, the screw measures M5 x 0.5 in accordance with SMS 1701, and the distance between the U-legs prior to cutting the thread is 4.5 mm. The straight cut threads 30 on the U-leg suitably define an angle of 1.95° to the cross direction of the nut. The nut may suitably be made of a plastic material, such as Delrin.

In the illustrated embodiment, the motor 1 is built together with a conventional gearbox 9 (not shown), so as to obtain a total ratio of 108:1 with the gearing 2. When using a conventional disposable syringe (3 ml), the syringe will dispense 0.5 µl of fluid with each rotation of the motor. The screw has a pitch of 0.5 mm, and 1/108th rotation of the motor will result in a translation of about 4 µm. Since the motor always rotates in one and the same direction, any play that is present will have only a negligible effect.

When using a motor designed for a nominal 12 V, it is possible to obtain a suitable feed speed with a 4 V battery, wherein one revolution of the motor will take about one-tenth of a second. When the device is used intermittently and one motor revolution is performed each minute, battery consumption will be so low as to enable a battery to last for fifteen calendar days. However, the investigation period is seldom longer than three calendar days.

As before mentioned, when inserting a microdialysis catheter, it is necessary to undertake a filling and flushing period, which, e.g., may involve the discharge of 100 µl of fluid at a rate of 15 µl per minute and thus with one motor revolution each other second. Furthermore, all transmission plays and clearances will be levelled out during this time period. In the following sampling period, fluid is discharged at one revolution per minute, thus at a quantity of 0.5 µl intermittently.

This example relates to a microanalysis catheter having an effective volume of 0.5 µl or negligibly thereabove. When other microdialysis catheters are used, the pump must be adapted accordingly, since the concept is to replace the fluid in the active part of the catheter on each feed occasion, with no surplus fluid being pressed through and therewith diluting the sample solution. Although having no part of the present invention, it can be mentioned that individual sample volumes are normally collected during successive time periods, for instance time periods of 15 min., 30 min. or 1 hr., depending on the speed at which, for instance, metabolic developments change, such as the changes in the sugar values of diabetics or other changes. In other cases, the equipment is used to deliver substances to a patient, and this example should not therefore be considered all-inclusive.

It is pointed out that the arrangement shown in the cut-away view of Fig. 4 is roughly to scale, and it will therefore be readily seen that the arrangement can be carried by a patient, for instance as shown in Fig. 2, and that the patient will experience no appreciable discomfort and that the arrangement can be used in any situation whatsoever and even when taking working samples. The person skilled in this art will understand that the device can be attached to the body of the patient with the aid of a strap or with the aid of adhesive tape.

## Claims

1. A microdialysis device comprising an elongated microdialysis catheter (A) having a semi-permeable membrane included in a holder which, together with the membrane, forms an outer tube in which an inner tube is housed, which has an opening at the distal end of the catheter, wherein a first line is connected to the proximal end of the inner tube and a second line is connected to a space formed between the inner tube and the outer tube at the proximal part of said space, a pump (B) which is connected to one of said lines and which delivers dialysis fluid, and means (C) for collecting from the end of the other one of said last-mentioned lines dialysis fluid that has flowed through the microdialysis device, **characterized in that** the pump is constructed to deliver a predetermined amount of fluid intermittently on each intermittent occasion, this amount being at most approximately equal to a volume that can be taken up in the space located between the inner tube (A2) and the outer tube (A1) along that part of the space which corresponds to the extension of the semi-permeable membrane in the longitudinal direction of the microdialysis catheter.

2. A device according to Claim 1, **characterized in that** the pump includes a casing (20) which houses a battery (7), an electric motor (1), gearing, a screw (3) which is connected to the screw via said gearing, and a replaceable injection syringe (6) comprising a cylinder and a plunger which moves in said cylinder and has a plunger rod which protrudes therefrom, a screw-threaded open nut means which can be brought into engagement with the screw at the distal end of said plunger, and a motor control means for advancing the plunger, preferably intermittently.

3. A device according to Claim 2, **characterized in that** a transmission means is provided between the motor (1) and the screw (3); and **in that** the motor control means includes a sensor (10, 11, 12) which senses rotation of the motor.

4. A device according to Claim 2 or Claim 3, **characterized in that** the transmission means is such that one motor revolution corresponds to a pump feed in the order of some tenths of a microliter.

## Patentansprüche

1. Mikrodialysevorrichtung, umfassend einen länglichen Mikrodialysekatheter (A) mit einer teildurchlässigen Membran, enthalten in einem Halter, der zusammen mit der Membran eine äußere Röhre bildet, in der eine innere Röhre aufgenommen ist, die an dem distalen Ende des Katheters eine Öffnung aufweist, wobei eine erste Leitung mit dem proximalen Ende der inneren Röhre verbunden ist und eine zweite Leitung mit dem proximalen Abschnitt eines Raumes, der zwischen der inneren Röhre und der äußeren Röhre gebildet ist, in Verbindung steht, eine Pumpe (B), die mit einer dieser Leitungen verbunden ist und eine Dialyseflüssigkeit fördert, und Mitteln (C) zum Sammeln von Dialyseflüssigkeit von dem Ende der anderen der zuletzt erwähnten Leitungen, welche durch die Mikrodialysevorrichtung geströmt ist,
**dadurch gekennzeichnet, dass**.
die Pumpe gestaltet ist, um eine vorbestimmte Menge an Flüssigkeit zu jeder diskontinuierlichen Gelegenheit diskontinuierlich zu fördern, welche Menge höchstens ungefähr gleich einem Volumen ist, das in dem zwischen der inneren Röhre (A2) und der äußeren Röhre (A1) gebildeten Raum, entlang dem Abschnitt des Raumes, der der Verlängerung der teildurchlässigen Membran in der Längsrichtung des Mikrodialysekatheters entspricht, aufgenommen werden kann.

2. Vorrichtung nach Anspruch 1, **dadurch gekennzeichnet, dass** die Pumpe ein Gehäuse (20), welches eine Batterie (7), einen Elektromotor (1), ein Getriebe, eine Schraube (3), die mit der Schraube via dem Getriebe verbunden ist, beherbergt und eine auswechselbare Injektionsspritze (6) beinhaltet, umfassend einen Zylinder und einen Kolben, der sich in dem Zylinder bewegt und eine Kolbenstange aufweist, die davon vorsteht, ein offenes Schraubengewinde Muttermittel, welches mit der Schraube an dem distalen Ende des Kolbens in Eingriff gebracht werden kann, und ein Motorsteuermittel, um den Kolben vorzugsweise diskontinuierlich vorzuschieben.

3. Vorrichtung nach Anspruch 2, **dadurch gekennzeichnet, dass** ein Getriebemittel zwischen dem Motor (1) und der Schraube (3) vorgesehen ist, und dadurch, dass das Motorsteuermittel einen Sensor (10, 11, 12) umfasst, welcher die Rotation des Motors abtastet.

4. Vorrichtung nach Anspruch 2 oder 3, **dadurch gekennzeichnet, dass** das Getriebemittel derart ausgestaltet ist, dass eine Motorumdrehung einer Pumpenspeisung in der Größenordnung von einigen Zehnteln eines Mikroliters entspricht.

## Revendications

1. Dispositif de microdialyse comprenant un cathéter de microdialyse allongé (A) ayant une membrane semi-perméable comprise dans un support qui, conjointement avec la membrane, forme un tube externe dans lequel est logé un tube interne, qui a une ouverture à l'extrémité distale du cathéter, dans laquelle une première ligne est reliée à l'extrémité proximale du tube interne et une deuxième ligne est reliée à un espace formé entre le tube interne et le tube externe sur la partie proximale dudit espace, une pompe (B) qui est reliée à l'une desdites lignes et qui délivre un fluide de dialyse, et des moyens (C) pour recueillir depuis l'extrémité de l'autre desdites lignes précitées un fluide de dialyse qui s'est écoulé à travers le dispositif de microdialyse, **caractérisé en ce que** la pompe est construite pour délivrer une quantité prédéterminée de fluide de façon intermittente à chaque occasion intermittente, cette quantité étant au moins approximativement égale à un volume pouvant être reçu dans l'espace situé entre le tube interne (A2) et le tube externe (A1) le long de cette partie de l'espace correspondant à l'extension de la membrane semi-perméable dans le sens longitudinal du cathéter de microdialyse.

2. Dispositif selon la revendication 1, **caractérisé en ce que** la pompe comprend un boîtier (20) qui loge une batterie (7), un moteur électrique (1), un engrenage, une vis (3) qui est reliée à la vis au moyen dudit engrenage, et une seringue à injection remplaçable (6) comprenant un cylindre et un piston qui se déplace dans ledit cylindre et a une tige de piston qui en fait saillie, des moyens formant écrou ouvert à filetage de vis qui peuvent être amenés en engagement avec la vis à l'extrémité distale dudit piston, et des moyens de commande du moteur pour faire avancer le piston, de préférence de façon intermittente.

3. Dispositif selon la revendication 2, **caractérisé en ce que** des moyens de transmission sont prévus entre le moteur (1) et la vis (3) ; et **en ce que** les moyens de commande du moteur comprennent un détecteur (10, 11, 12) qui détecte la rotation du moteur.

4. Dispositif selon la revendication 2 ou la revendication 3, **caractérisé en ce que** les moyens de transmission sont tels qu'un tour du moteur correspond à l'alimentation d'une pompe de l'ordre de quelques dixièmes de microlitre.
